Europäisches Patentamt

**European Patent Office**

**Office européen des brevets**

(11) Numéro de publication: **0 011 594**
**B1**

(12) **FASCICULE DE BREVET EUROPEEN**

(45) Date de publication du fascicule du brevet: **05.12.84**

(21) Numéro de dépôt: **79810067.3**

(22) Date de dépôt: **07.08.79**

(51) Int. Cl.³: **G 06 F 15/02, A 61 B 10/00**

(54) Calculatrice de poche pour la prévision d'événements périodiques comprenant une mémoire électromécanique.

(30) Priorité: **09.08.78 CH 8455/78**

(43) Date de publication de la demande:
**28.05.80 Bulletin 80/11**

(45) Mention de la délivrance du brevet:
**05.12.84 Bulletin 84/49**

(84) Etats contractants désignés:
**AT BE CH DE FR GB IT LU NL SE**

(56) Documents cités:
**DE-A-2 537 400**
**DE-A-2 652 994**
**FR-A-1 413 757**
**FR-A-2 317 705**
**US-A-4 059 952**

**Technische Rundschau, no. 12, 21.3.78, pp. 5,7**
**Hewlett-Packard Journal, vol. 27, no. 3, pp. 2à7, novembre 1975**
**Elektronik 1975, Heft 2, pp. 56-60**
**IBM Technical Disclosure Bulletin, vol. 19, no. 7, décembre 1976. pp. 2419-2423**

(73) Titulaire: **Bioself International Inc.**
**P.O. Box 10496 Shirley Street 50**
**Nassau, Bahamas (BS)**

(72) Inventeur: **Desjacques, Edmond**
**91, rue de Genève**
**CH-1226 Thônex GE (CH)**

(74) Mandataire: **Kirker, Gaylord Emile**
**c/o KIRKER & Cie S.A. 14, Rue du Mont-Blanc**
**Case postale 872**
**CH-1211 Genève 1 (CH)**

Courier Press, Leamington Spa, England.

## Description

On sait réaliser des calculatrices miniatures capables d'effectuer des calculs relativement complexes.

On connaît d'autre part des méthodes permettant à une femme de prévoir, avec une marge donnée, les périodes où un rapport sexuel sera probablement suivi d'une grossesse. Une telle méthode est la méthode Ogino. La réalisation d'une calculatrice miniature affectée à la prévision des périodes selon une méthode ou une autre n'offre en principe aucune difficulté. Toutefois il se pose un grave problème de la conservation des données de base. En effet, les calculatrices courantes sont conçues de telle sorte que l'utilisateur doit fournir les données au moment où il désire résoudre un problème, et préciser la nature des calculs, la machine n'ayant en mémoire que des séquences d'ordres correspondant à des opérations de base et quelques constantes de base. D'habitude la calculatrice n'est pas conçue pour retenir des données. Or, si l'on veut effectuer une prévision, il est indispensable de disposer des dates de quelques périodes antérieures. Pour effectuer une prévision avec une marge de sécurité raisonnable, on tiendra compte, par exemple, d'une moyenne portant sur les quatre dernières périodes.

Ces données sont importantes et elles doivent être conservées de moins en mois. On ne peut évidemment pas utiliser, pour ces données, un circuit de mémoitre usuel qui s'efface lorsque l'alimentation est coupée.

Pour certaines calculatrices, on fait usage de cartes magnétiques miniatures. Toutefois un tel moyen ne représente pas ici une sécurité absolue. En effet, la carte magnétique peut être égarée et les données qu'elle contient peuvent se trouver effacées ou altérées par l'influence d'un champ magnétique entrant en contact avec la carte.

La présente invention vise à fournir une solution commode et sûre au problème de la conservation de quelques données essentielles, notamment pour le cas d'une calculatrice de poche servant à la prévision des cycles menstruels.

La calculatrice selon l'invention fait l'objet de la revendication 1, les autres revendications portant sur des formes d'exécution spéciales.

La calculatrice selon l'invention présente une solution commode aux personnes désirant utiliser une méthode de prévision telle que la méthode Ogino. Les données essentielles sont mémorisées dans une mémoire spéciale incorporée à la calculatrice et non dans un support mémoire externe du genre carte magnétique. On ne risque donc pas de l'égarer.

De plus, la mémoire ainsi incorporée à la calculatrice ne court pratiquement aucun risque de se trouver directement en contact avec un aimant qui pourrait la perturber, et la mémoire est ainsi beaucoup plus sûre qu'une mémoire sur carte magnétique.

Enfin, si l'on choisit un type convenable d'élément électro-mécanique, tel qu'il est décrit dans FR—A 1413757, le mémoire électro-mécanique présentera une bonne immunité aux influences extérieures telles que chocs, variations de température et de pression. Notamment, un type préféré d'élément électro-mécanique est protégé contre une accélération de plusieurs dizaines de G.

La calculatrice peut être réalisée de manière que son emploi soit des plus simples et ne demande à l'utilisateur qu'un minimum de raisonnement et d'attention. A l'inverse, elle peut être associée à une calculatrice relativement complexe et/ou à une horloge digitale.

Parfois, la personne, pour des raisons physiologiques, pourra avoir besoin d'un programme de prévision différent d'un programme standard et fonction, par exemple, des prescriptions d'un gynécologue. A cette fin, on peut prévoir une forme de réalisation particulière où la mémoire supportant le programme comporte une partie amovible ou reprogrammable. Par exemple, le bloc mémoire de la calculatrice peut consister en une mémoire ROM comportant le programme de prévision standard et remplaçable par une mémoire différente, programmée sur la base des prescriptions du gynécologue; ou bien, le bloc mémoire comporte une mémoire EPROM, c'est-à-dire effaçable, en principe à l'aide de rayons ultraviolets intenses, et reprogrammable à l'aide d'un appareil auxiliaire que pourrait détenir le cynécologue ou la société distribuant la calculatrice.

Une calculatrice selon l'invention peut donc avantageusement être appliquée à la prévision de cycles menstruels de la femme, notamment selon la méthode Ogino; cependant, les principes à la base de l'invention peuvent s'appliquer à tout problème que l'on peut soumettre à une calculatrice miniature et du genre où l'on désire conserver, d'une époque à une autre, un certain nombre de données essentielles qui demandent une mémoire sûre, incorporée à la calculatrice et conservant les informations indépendamment de l'alimentation électrique.

La calculatrice selon l'invention pourrait avantageusement être adaptée au calcul des biorythmes. On connaît une calculatrice de biorythme à fonctionnement essentiellement électronique. Toutefois cette calculatrice connue présente l'inconvénient d'une mémoire volatile, c'est-à-dire dont les données sont perdues lorsqu'on coupe l'alimentation. La calculatrice selon l'invention permettrait, dans un tel cas, de remédier à cet inconvénient et de conserver en mémoire, même si l'alimentation est coupée, une donnée de calcul telle que la date de naissance, la mémoire étant à lecture et écriture électriques, et utilisable dès que l'alimentation est rétablie.

On connaît par le CH—A 578.766 une calculatrice de biorythme où l'inscription des courbes est effectuée à l'aide d'organes électro-mécaniques. Si l'alimentation est coupée, les roues
dentées conservent leur position de sorte que
l'inscription des courbes pourrait être achevée
après rétablissement de l'alimentation. Mais
comme on le voit, ceci ne constitue pas une
véritable mémoire qui peut être relue par un
circuit de calcul et rien n'est prévu pour
mémoriser une donnée telle que la date de naissance dans une mémoire électro-mécanique
conservant la donnée même si l'alimentation
est coupée, par exemple entre deux utilisations. La calculatrice décrite dans ce brevet
ne saurait donc se comparer à la présente
invention.

La présente invention pourrait également
être avantageusement adaptée à un réveil
digital pour conserver l'heure de l'actionnement de la sonnerie, même au cas où l'alimentation est coupée.

On pourrait également mémoriser de la sorte
des dates, pour le cas où le réveil comporte un
calendrier électronique et des moyens pour
signaler l'imminence ou l'occurrence de la date
que l'utilisateur a inscrite dans la mémoire du
réveil. Ces moyens de signalement peuvent être
par exemple un clignotement d'une partie de
l'affichage. La solution selon l'invention permettrait, par exemple, de changer les piles sans
crainte que la date importante ou l'heure notée
ne soit perdue ou altérée.

Ce qui précède montre l'intérêt de la
présente invention dans le domaine les calculatrices électroniques miniatures affectées à la
résolution de certains problèmes pour lesquels
il est souhaitable de conserver des données de
manière commode et fiable même si l'alimentation est coupée.

L'invention concerne également le domaine
des dispositifs miniatures électroniques qui ne
sont pas de pures calculatrices mais qui, cependant, comme par exemple un réveil digital, comprennent une ou plusieurs fonctions utilisant
des données, par exemple l'heure de réveil,
données qu'il est souhaitable de conserver de
manière commode et fiable, même si l'alimentation est coupée.

L'invention sera mieux comprise à l'aide de la
description de quelques forms de réalisation.
données ci-après à titre d'exemple et en référence aux dessins.

La fig. 1 représente une forme de réalisation
de la calculatrice selon l'invention.

La fig. 2 représente de manière schématique
les circuits de la calculatrice de la fig. 1.

La fig. 3 représente le schéma d'une
mémoire à éléments électro-mécaniques.

La fig. 4 représente un élément de mémoire
électro-mécanique.

La fig. 5 illustre une autre forme de réalisation de la calculatrice.

La fig. 6 illustre une troisième forme
d'exécution de la calculatrice.

Les fig. 7A, B et C illustrent les états d'un
élément spécial d'affichage.

Les fig. 8A, B et C illustrent divers contenus
possibles de l'affichage, et

La fig. 9 représente de manière schématique
les circuits de la calculatrice de la fig. 6.

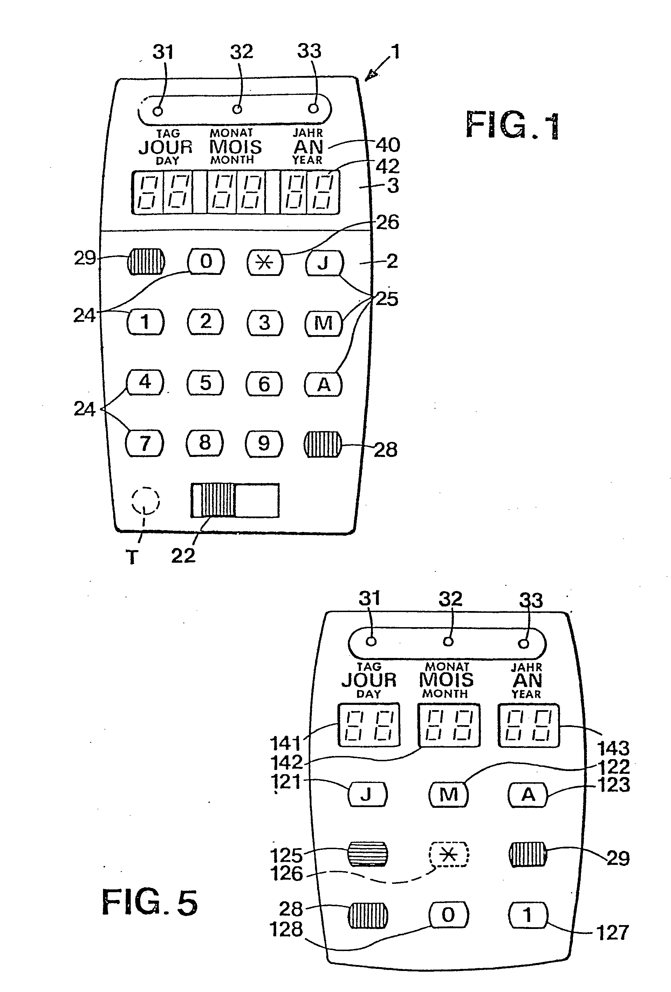

La calculatrice représentée à a la fig. 1
comporte un boîtier 1 renfermant les circuits et
sur lequel sont disposés des organes de
commande et de lecture.

Les organes de commande consistent en un
clavier 2 comportant une touche ON/OFF 22
pour la mise en service et l'arrêt, des touches
numériques telles que la touche 24, des
touches de fonction 25 pour indiquer la nature
de la donnée fournie, jour, mois, année, une
touche d'ordre 26 pour demander une prévision, et une touche d'inscription 29, munie
d'une touche de sécurité 28 telle que
l'inscription ne se fait que si l'on appuie simultanément sur les touches 28 et 29.

La calculatrice comprend également un
affichage comprenant des diodes électroluminescentes verte 31, jaune 32 et rouge 33.
Ces diodes sont destinées à indiquer la probabilité qu'un éventuel rapport intime soit suivi
d'une grossesse selon la convention suivante:
lumière rouge, probabilité élevée; lumière jaune,
probabilité moyenne; lumière verte, probabilité
faible.

L'affichage comporte également une partie
digitale comprenant au moins six cellules 42 à
sept segments, du type soit électroluminescent, soit à cristaux liquides, pour
afficher numériquement le jour, le mois et
l'année (unités et dizaines du numéro de l'année
civile).

Les inscriptions 40 telles que "JOUR",
"MOIS", "AN" facilitent la lecture de la date.

Les fonctions logiques nécessaires sont en
principe réalisables à l'aide de circuits tels que
portes, registres, compteurs, etc. En pratique
toutefois, on partira de circuits plus évolués du
genre microprocesseur, plus souples d'emploi,
et plus commodes à implanter dès que les
fonctions à réaliser dépassent le niveau des
fonctions élémentaires telles qu'un comptage
simple ou un transfert conditionnel.

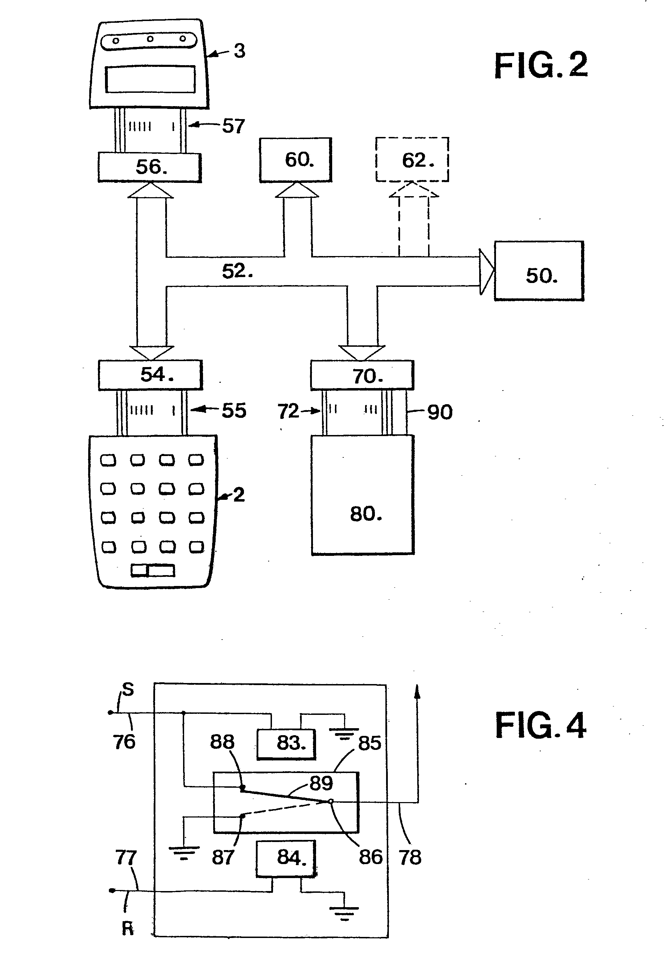

Le schéma de la fig. 2 comprend un système
logique miniature organisé autour d'une unité
de processeur central CPU 50, contrôlant des
mémoires et des périphériques par l'intermédiaire d'un bus 52. Comme périphérique, on
trouve le clavier 2 relié par des lignes 55 à un
circuit d'interface 54 relié au processeur CPU
50 par le bus 52. Comme périphérique, on
trouve également l'affichage 3 relié par des
lignes 57 à un circuit d'interface 56 relié au bus
52.

Le programme destiné à être exécuté par le
processeur est logé dans une mémoire ROM 06,
c'est-à-dire destinée à être lue seulement. Une
telle ROM peut en fait consister en une PROM,
c'est-à-dire une mémoire ROM que l'on peut, à
l'aide d'appareils auxiliaires, programmer à
volonté. Cette mémoire est de préférence
implantée sous la forme d'un "chip" amovible, c'est-à-dire un circuit miniature pourvu de broches destinées à se connecter dans un socle pourvu de connecteurs correspondants. Un chip amovible peut être sorti être modifié ou remplacé. On peut utiliser une EPROM, c'est-à-dire un circuit effaçable, en principe à l'aide de rayons ultraviolets intenses, et reprogrammable à l'aide d'un appareil auxiliaire ad hoc.

Les calculs font usage de variables auxiliaires et de données en provenance du clavier qui doivent être mémorisées dans une mémoire RAM 62. Toutefois, si le nombre de ces variables n'est pas trop important, on peut considérer que le processeur comporte les moyens voulus pour les mémoriser. Les processeurs, connus comportent usuellement une pluralité de registres destinés à cet effet.

Ainsi, la calculatrice vise à réaliser un certain nombre de fonctions pour résoudre le problème posé dans l'introduction. Pour ce faire, on utilise un microprocesseur commandant un certain nombre de périphériques comme expliqué ci-dessus, au moyen d'un programme. N'importe quelle personne qualifiée dans le domaine des microprocesseurs est capable de réaliser ce programme logé dans la mémoire ROM 60 en langage "ASSEMBLEUR", en fonction du problème posé. Cette personne utilisera par exemple un microprocesseur du type "6500", des mémoires ROM et RAM du type "8 bits" et des interfaces du type "6520", tous fabriqués par exemple chez "Synertec" ou "Intel", ces entreprises délivrant également les manuels d'utilisation y relatifs décrivant exactement le fonctionnement de ces dispositifs.

Il existe également des "chips" incorporant à la fois une mémoire PROM, une portion de mémoire EPROM, une mémoire RAM et le processeur central CPU lui-même. Le principe du schéma reste le même, mais un tel circuit peut présenter l'avantage d'un encombrement réduit. On se référera de préférence au modèle TMS 1000 de TEXAS INSTRUMENTS.

Sur le schéma de la fig. 2, on trouve encore une mémoire 80 à éléments électro-mécaniques reliée à une circuit d'interface 70 par des lignes 72 et 90. Une telle mémoire présente des particularités liées aux principes à la base de la présente invention. Cette mémoire est également contrôlée par le microprocesseur programmé à cet effet.

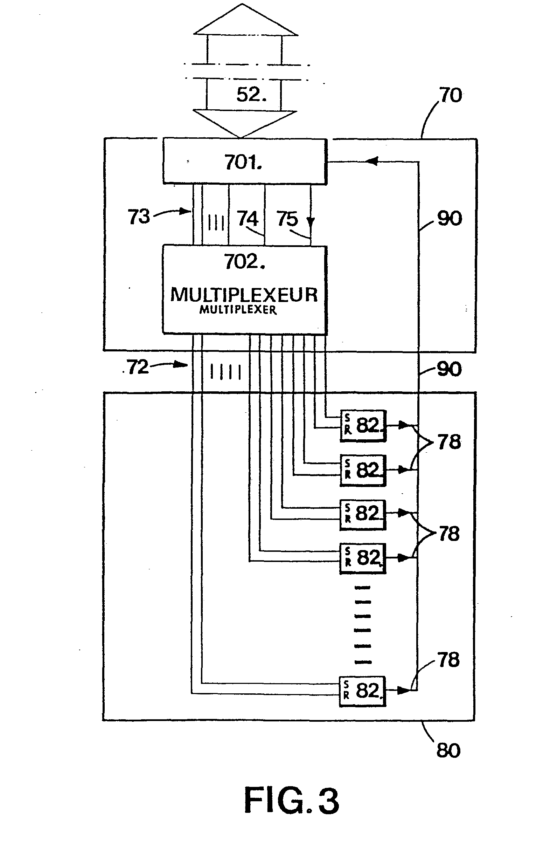

La fig. 3 montre le schéma de la mémoire électro-mécanique 80 et de son interface 70.

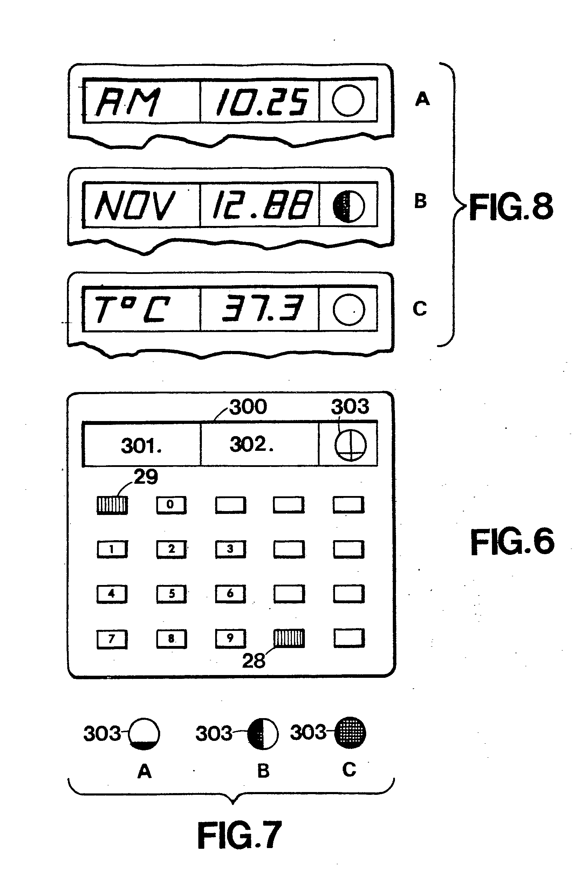

La mémoire se compose d'une pluralité d'éléments de mémoire électro-mécaniques 82. Chaque élément 82 peut être assimilé à une bascule, pouvant donc mémoriser une information de 1 bit, et l'élément sera décrit plus en détail à la fig. 4.

Chaque bascule comprend une entrée S (set) de mise à 1 et une entrée R (reset) de mise à zéro, et un fil de sortie 78, tous les fils de sortie étant reliés en un fil de sortie unique 90 pour toute la mémoire 80.

Les entrées R et S de chaque élément 82 sont reliées individuellement à l'une des sorties d'un multiplexeur 702. Le choix de la sortie est commandé par les fils de commande 73 du multiplexeur et le fil 74 sert à définir si on veut un R ou un S. Lorsque le multiplexeur a sélectionné la sortie appropriée en fonction de l'adresse (fils 73) et de la donnée (fil 74), une impulsion de commande peut être envoyée par le fil et elle sera aiguillée par le multiplexeur vers l'entrée choisie.

L'impulsion d'inscription R ou S doit avoir une certaine puissance et une certaine durée, par exemple 10 milliampères pendant 2 millisecondes, pour exciter les organes électromécaniques de l'élément 82.

Pour lire, on envoie une impulsion également par le multiplexeur sur l'entrée S de l'élément 82 qu'on veut lire, mais on envoie une impulsion beaucoup plus courte, par exemple 10 microsecondes. Cette impulsion ne peut pas altérer l'état logique de la bascule. A ce moment, si la bascule est à 1, la sortie 78 et le fil de donnée 90 fourniront un 1 logique et si la bascule choisie est à zéro, la sortie 78 et le fil de donnée 90 resteront à 0, de sorte qu'on dispose, pendant 10 microsecondes, de la valeur logique de la bascule choisie sur le fil de donnée 90, ce qui suffit pour la lecture.

Le multiplexeur 702 est lui-même commandé par le microprocesseur au travers d'un amplificateur 701, le microprocesseur gérant les inscriptions et les lectures dans la mémoire 80. L'amplificateur 701 est évidemment en communication à double sens au moyen du bus 52 avec le microprocesseur 50 dont il dépend. Il peut être simplement constitué d'un circuit amplificateur à transistors. Le circuit 701 et le multiplexeur 702 peuvent être considérés comme formant l'interface de la mémoire 80.

La fig. 4 représente le schéma de l'élément de mémoire électro-mécanique 82. L'élément comporte un contact bistable 85 comprenant une lame 89 reliée à une borne 86 et établissant le contact soit avec la borne 88, comme sur la figure, soit avec la borne opposée 87. La commutation est commandée par deux bobinages 83 et 84. Une impulsion sur le bobinage 83 fait venir la lame 89 dans la position représentée à la fig. 4 et une impulsion sur l'autre bobinage 84 fait venir la lame dans la position opposée, indiquée en pointillé. Une bobine est reliée à l'entrée S et l'autre bobine est reliée à l'entrée R. De plus, l'entrée S est reliée à la borne 88, pour permettre les lectures.

La matrice des mémoires 80 représentée à la fig. 3 est réalisable sous la forme d'une plaque de circuit imprimée portant une pluralité de relais bistables miniatures 82 soudés à la plaque par leurs bornes de contact. Un modèle de relais pouvant convenir est le relais de la série TL fournie par la Compagnie Deutsch. Un tel relais résiste à des conditions telles qu'une vibration de 3000 Hz présentant une accélération de 30 g ou une accélération de 100 g sur 6 millisecondes. Son encombrement est de l'ordre d'un centimètre cube ou moins de sorte qu'on peut aisément en loger plusieurs dizaines dans le boîtier d'une calculatrice en restant dans des dimensions acceptables pour une calculatrice de poche. Sur le marché, on trouve de tels relais. On utilisera de préférence des relais de la série "TL" de la Compagnie Deutsch.

Avant de poursuivre la description, il importe de rappeler approximativement en quoi consiste la méthode connue pour la prévision.

La personne note les dates de ses règles successives. On calcule la durée, en nombre de jours, entre deux dates consécutives. Lorsque cette durée est régulière à quelques jours près pour plusieurs cycles successifs et que cette durée est comprise entre environ 21 et 37 jours, on peut raisonnablement appliquer la méthode qui part du principe que l'ovulation a lieu 13 jours avant le début des règles. La probabilité qu'un rapport sexuel soit suivi d'une grossesse est alors maximale au moment de l'ovulation. Cette probabilité est également élevée pour les quatre jours précédents et les trois jours suivants.

Cette probabilité devient ensuite quasi nulle jusqu'aux prochaines règles. Le reste du temps il subsiste une probabilité de grossesse relativement réduite.

Un exemple de méthode consiste donc à noter les dates des règles successives $d_1, d_2, d_3, \ldots$, à calculer les durées correspondantes des cycles $C_1, C_2, C_3, \ldots$, à vérifier qu'ils sont réguliers, à établir la moyenne cm et l'erreur probable ec. On calcule la date probable dp des prochaines règles. Cette date vaut, bien sûr, la date des dernières règles dn, plus la durée moyenne du cycle cm, avec une incertitude em:

$$dp=dn+cm\pm em$$

On calcule combine de jours il y a entre la date d'aujourd'hui, da, et la date probable, dp. Soit g, ce nombre de jours: $g=dp-da$. Si g est compris entre $14+4+ec$ et $14-3-ec$, alors la probabilité d'une grossesse subséquente est élevée. Si g est supérieur à $14+ec+4$, la probabilité est moyenne. Si g est inférieur à $14-ec-4$, alors la probabilité est faible.

Concernant la calculatrice, on voit que les données importantes à mémoriser sont: la date du début des dernières règles et les durées des quatre derniers cycles pour pouvoir établir une moyenne. La mémorisation d'une date implique une portion de mémoire pouvant enregistrer un nombre j ayant une valeur arbitraire entre 1 et 365, pour le jour dans l'année, ainsi qu'un nombre "a" compris entre 0 et 99 pour désigner l'année.

La date requiert ainsi 9 bits pour j et 7 bits pour a. Quant aux durées des cycles c, on les considère comme valables seulement si elles sont comprises entre 22 et 37. On enregistre alors seulement la différence dc entre la durée du cycle c et 22:

$$dc=c-22$$

Comme c vaut au plus 37, dc vaut au plus 15:

$$15=37-22.$$

dc est donc compris entre 0 et 15, ce quie requiert 4 bits. Comme on enregistre 4 périodes, cela demande $4\times4=16$ bits. La capacité de la mémoire 80 doit être au minimum donc de 16 bits pour les durées de cycles, plus 7 pour a, soit en tout 32 bits.

Si pour chaque période on prend cinq bits et non quatre, on peut bien entendu couvrir une plage de valeurs ayant une extension double, c'est-à-dire pour un dc allant de 0 à 31. Dans ce cas on pourra calculer la différence dc à partir d'une limite inférieure qui serait plus basse que 22, et aller à une limite supérieure plus élevée que 37. La plage des valeurs pour les durées c pourrait être comprise par exemple entre 14 et 45. Bien entendu, le programme devra tenir compte de ce que les valeures extrêmes ne permettent pas d'appliquer la méthode et faire apparaître sur l'affichage un signal indiquant cela.

L'utilisation et le fonctionnement vont maintenant être décrits en référence aux fig. 1 et 2 particulièrement.

On suppose que la mémoire 80 contient la date des dernières règles ainsi que les valeurs (diminuées de 22) des durées des quatre derniers cycles, que l'on se trouve dans des conditions où la méthode est applicable et que l'on est en mai 1988.

L'intéressée désire obtenir la prévision pour le lendemain, qui est le 20 du mois. A cet effet, elle actionne sur le clavier les touches portant les symboles selon la séquence suivante:

J20M05A88*

ce qui veut dire: jour: 20; mois: 5; année: 88; ordre de calculer (touche 26). Le processeur 50 commande l'affichage de ces nombres dès que la personne les inscrit au clavier. Le processeur 50 calcule la durée moyenne cm du cycle sur la base des quatre derniers cycles, l'erreur probable ec, le nombre g de jours entre cette date probable et la date du 20, en consultant bien sûr la mémoire 80, sans l'altérer.

Le processeur évalue, d'après g, cm et em, si la probabilité est élevée, moyenne ou faible et, selon le résultat, allume l'une des trois diodes rouge 31, jaune 32 ou verte 33, respectivement.

Concernant le calcul des intervalles de temps entre différentes dates, la calculatrice met en oeuvre des moyens analogues à ceux mis en oeuvre dans certaines calculatrices financières de poche, qui sont dotées de ce qu'on appelle un calendrier perpétuel électronique. Dans le cas de la calculatrice décrite ici, les dits moyens comprendront simplement un programme adéquat et quelques données fixes, toutes inscrites dans une portion de la mémoire ROM 60 qui est exploitée par le processeur 50.

Bien entendu, le programme possède en outre, de préférence, des routines permettant de déceler les cas où on ne peut pas établir de prévision raisonnable, par exemple parce que la date pour laquelle on veut la prévision est trop éloignée ou impossible, ou parce que les cycles sont trop irréguliers ou trop courts. De préférence, le programme prévoit alors l'affichage de la lettre E, pour "erreur" suivie éventuellement d'un numéro indiquant la nature de l'erreur, permettant à la personne de se référer à la notice d'emploi généralement jointe à la calculatrice ou imprimée au dos de la calculatrice.

Bien entendu, il conviendrait de renseigner les acquéreurs pour éviter qu'ils n'attribuent à la calculatrice des possibilités exagérées.

Concernant la modification des données, elle intervient lorsque la personne inscrit la date de ses dernières règles. Si elles interviennent, par exemple, le 28 mai 1988, la personne actionnera les touches

J28 M05 A88,

elle vérifie sur l'affichage que la date est bien correcte puis elle actionne simultanément la touche d'enregistrement 29 et la touche de sécurité 28.

Ceci actionne un programme de mise à jour des données de la mémoire 80. Ce programme calcule la dernière durée de cycle par différence entre la date des règles précédentes encore inscrite en mémoire 80, et la date des règles récentes, qui vient d'être fournie par le moyen du clavier 2. Il élimine, parmi les quatre durées de cycles mémorisées celles qui concernent le cycle le plus ancien, décale les trois autres et inscrit la durée du cycle le plus récent. Le programme remplace encore la date des règles par la dernière date.

Bien entendu, la mémoire 80 comporte de préférence un nombre d'éléments supérieurs au minimum requis, qui est de 32 comme on l'a vu, pour être à même d'inscrire avant d'effacer afin qu'on ne perde pas les données au cas où l'alimentation est coupée pendant le transfert. La mémoire comportera en outre des bits d'état pour indiquer si l'opération d'enregistrement est achevée ou inachevée, pour faciliter la récupération des données au cas où l'alimentation est coupée pendant le transfert.

De plus, pour les quatre durées de cycle, la mémoire peut comporter un pointeur qui dit lequel des quatre est le plus récent, étant entendu que les autres sont ordonnés selon un "stack" circulaire. Deux bits suffisent pour le pointeur.

La méthode Ogino peut être complétée par un test concernant la température. Le principe est suffisamment décrit dans la littérature médicale.

Une variante de la calculatrice peut alors comprendre un moyen pour l'introduction de la donnée relative à la température et des programmes pour compléter les calculs par une fonction tenant compte de ces données. Comme moyen d'entrée, une touche supplémentaire suffit. Soit T une telle touche supplémentaire.

L'utilisation pourrait être la suivante: lorsque la personne demande une prévision, elle inscrit au clavier non seulement la date, mais encore la température, par exemple 37,3°C, avant de demander la prévision en actionnant la touche 26. La séquence serait:

J20M05A88

puis, après vérification sur l'affichage: T373 puis * pour ordonner le calcul d'une prévision.

Concernant l'inscription d'une date au clavier, il est bien clair que l'on pourrait inscrire le jour, le mois et l'année dans un order différent. De plus, si on se rend compte que l'une des indications, par exemple le mois, est erronée, il suffit d'actionner à nouveau la touche M puis les touches numériques correctes.

On peut encore remarquer que la touche * 26 peut être éliminée. Dans ce cas, le programme est agencé de manière à effectuer la prévision pour toute date que l'on inscrit et qui apparaît sur l'affichage. Ce qui précède concerne un exemple de réalisation et nombre de modifications peuvent être effectuées sans sortir du cadre de la présente invention.

La fig. 5 illustre une deuxième forme de réalisation qui se distingue par une certaine simplicité du clavier. Le système logique d'une telle calculatrice possède essentiellement la structure décrite à la fig. 2. Toutefois les programmes tiennent compte du clavier particulier à cette forme d'exécution.

L'utilisation et le fonctionnement sont décrits ci-après en même temps que l'on décrit les fonctions des différentes touches.

La touch "1" 127 sert à la mise en service, elle branche l'alimentation. La touche "0" 128 sert à l'arrêt et coupe l'alimentation. Toutefois, même si l'on n'actionne pas la touche "0", l'affichage est mis en veilleuse au bout d'une minute si l'on n'a actionné aucune touche du clavier. L'affichage est à nouveau activé si l'on presse à nouveau la touche 1 ou aussi si l'on actionne une autre touche, mais à l'exclusion de la touche 0 qui arrête tout. Si l'on n'actionne aucune touche pendant 5 minutes, le système est également mis à l'arrêt.

La touche J sert à modifier un compteur numérique dont le contenu est affiché sur les deux digits de la fenêtre 141. Le dit compteur numérique est évidemment de nature essentiellement logicielle (soft); il n'y a donc pas lieu de le décrire ici.

Si le nombre affiché est supérieur à la date du jour que l'utilisateur veut afficher, l'utilisateur peut utiliser la touche d'inversion 125, laquelle a pour effet d'inverser le sens de comptage.

Le programme est agencé pour que le comptage soit relativement lent lorsqu'on actionne la touche J, puis au bout de quelques secondes, le comptage accélère si l'on maintient la touche J.

La touche M 122 permet d'afficher le mois sur les digits de la fenêtre 142 et la touche A 123 permet d'afficher l'année sur les deux digits de la fenêtre 143. Les touches M et A ont des fonctions analogues à celle de la touche J et sont également influencées par la touche d'inversion 125.

Le clavier peut comporter une touche 126 ordonnant le calcul d'une prévision. On a vu qu'une telle touche n'est pas indispensable. Le clavier comporte enfin les touches d'inscription 28 et de sécurité 29 dont la fonction a déjà été décrite.

La fig. 6 illustre un exemple de réalisation qui, à l'inverse de celui de la fig. 5, se distingue par une certaine complexité. Le clavier comporte des touches numériques et diverses touches de fonction et pourrait parfaitement convenir pour une calculatrice scientifique, financière, arithmétique ou autre, ainsi qu'à une horloge électronique. Le clavier comporte en outre une touche d'inscription 29 et une touche de sécurité 28.

L'affichage 3 comprend, dans l'exemple donné, une zone alphanumérique 301 de trois positions, une zone numérique 302 de quatre positions et une zone spéciale 303 pour l'affichage du calcul de prédiction.

Une probabilité faible est affichée de la manière indiquée à la fig. 7A, une probabilité moyenne est affichée de la manière indiquée à la fig. 7B, une probabilité élevée est affichée comme indiqué à la fig. 7C et, en outre, par un clignotement de l'élément d'affichage spécial 303. L'affichage 300 peut être réalisé avantageusement, mais non exclusivement, selon la technique des cristaux liquides.

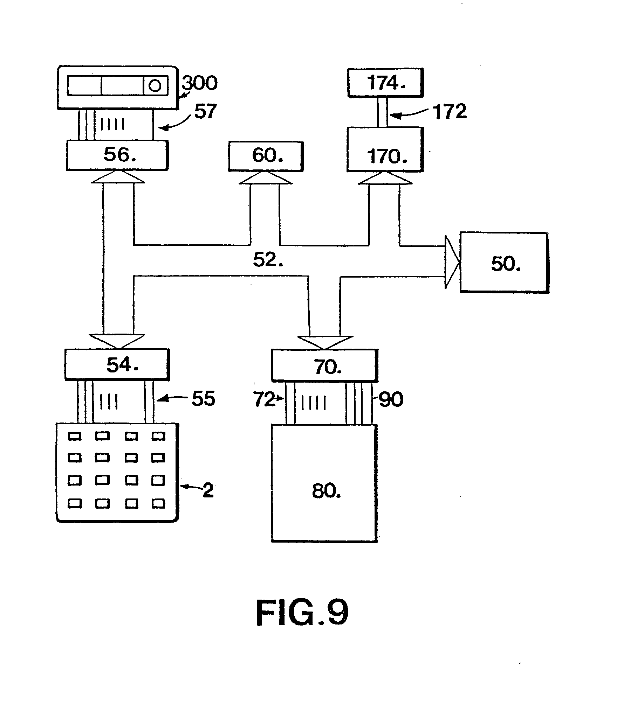

La fig. 9 montre le schéma d'une calculatrice comportant une partie servant à la mesure du temps. Cette partie comporte une base de temps 174 reliée à un ensemble de circuits logiques de mesure du temps 170 qui fonctionnent en permanence et, entre autres, évaluent non seulement l'heure à la seconde près, mais encore la date. Ces circuits 170, 174 sont destinés à fonctionner sans interruption.

Un tel circuit de mesure permanente du temps représente un moyen commode pour faire apparître, comme donnée, la date du jour. Cette date peut ensuite être exploitée pour un calcul de prévision ou une inscription.

La fig. 8 illustre quelques états possibles de l'affichage 300.

La fig. 8A illustre l'affichage de l'heure, 10h25 du matin dans l'exemple donné. La zone alphanumérique est utilisée pour distinguer le matin ("AM") de l'après-midi ("PM").

La fig. 8B illustre l'affichage d'une date, 12 novembre 1988 dans l'exemple donné, ainsi que l'affichage d'un calcul de prévision qui indique, dans l'exemple donné, une probabilité moyenne.

La fig. 8C indique un troisième emploi de l'affichage, prévu pour le cas où l'on peut fournir au programme de calcul de prévision une donnée concernant la température, qui est de 37,3°C dans l'exemple donné.

Les exemples décrits ci-dessus se réfèrent spécialement à une calculatrice agencée spécialement pour exécuter les calculs requis par la méthode Ogino ou une méthode analogue. Toutefois, le principe consistant à utiliser une mémoire électro-mécanique composée d'une pluralité de relais bistables et de circuits de lecture et d'enregistrement peut s'appliquer à d'autres calculatrices ou d'autres dispositifs électroniques comportant une partie digitale utilisant des données, pour le cas où certaines données importantes doivent être mémorisées de façon commode et sûre et conservées même en l'absence d'alimentation.

**Revendications**

1. Calculatrice de poche pour la prévision d'événements périodiques, comprenant: une alimentation électrique, des moyens (2) d'entrée permettant de communiquer des données comprenant des dates et des ordres de calcul, ces moyens comprenant au moins une sortie et délivrant à cette sortie les dites données et les dits ordres sous forme de signaux électriques, des moyens de calculs électroniques (50) comprenant des entrées pour les dits signaux, des circuits de calcul (50) et de mémoire (60, 62, 80) agencés pour effectuer les calculs de prévision en réponse à des ordres communiqués aux moyens d'entrée, et au moins une sortie (57) pour des signaux correspondant aux résultat demandés, et des moyens d'affichage (3) commandés par les signaux de sortie, caractérisée en ce qu'elle comprend en outre pour conserver des données valables essentielles, une mémoire électro-mécanique (80) formée d'unités de mémoire électro-mécaniques, chaque unité de mémoire électro-mécanique se présentant sous la forme d'un relais miniature électro-mécanique bistable (82) ayant au moins un solénoïde (83, 84) de commande et conservant sa position mécanique même en l'absence d'alimentation électrique, de manière à préserver les données variables essentielles même si l'alimentation est coupée, les contacts du relais servant à la lecture de l'unité mémoire (82) constituée par le relais, la mémoire (80) comprenant des circuits d'inscription (72) permettant de transmettre à volonté une impulsion à chaque solénoïde (82), et des circuits de lecture (78) permettant à volonté de tester l'état, ouvert ou fermé, de chaque contact, les dits circuits de lecture étant commandés par les moyens de calcul (50), et en ce que la mémoire électromécanique (80) est agencée pour être lue seulement par les moyens de calcul (50) et pour être mise à jour par introductions successives de données par actionnement manuel des moyens d'entrée (2), et en ce que, pour l'inscription de données dans la mémoire électromécanique, la calculatrice comporte une touche de commande (29) et une touche de sécurité (28) agencées de telle sorte que pour inscrire des données dans la mémoire électromécanique (80), il faut presser les deux touches (28, 29).

2. Calculatrice selon la revendication 1, caractérisée en ce qu'elle comporte une base de temps et des circuits électroniques logiques de mesure du temps, et en ce que les moyens de calculs et d'affichage comprennent des fonctions de mesure et d'affichage du temps.

3. Calculatrice selon la revendication 1 ou 2, caractérisée en ce que l'affichage comprend plusieurs éléments électro-optiques pour indiquer le résultat du calcul.

4. Calculatrice selon la revendication 1, caractérisée en ce que l'affichage comprend plusieurs éléments électro-luminescents de couleurs différentes.

5. Calculatrice selon l'une des revendications 1 et 2, caractérisée en ce que les moyens d'affichage comprennent un affichage numérique permettant de lire au moins les données que l'on introduit.

6. Calculatrice selon l'une des revendications précédentes, caractérisée en ce que les moyens d'affichage comprennent un affichage alphanumérique.

7. Calculatrice selon l'une des revendications précédentes, caractérisée en ce que la mémoire électro-mécanique (80) est reliée à un bus (52) central par un circuit d'interface (70).

8. Calculatrice selon la revendication 7, caractérisée en ce que le circuit d'interface (70) comprend un multiplexeur (702).

**Patentansprüche**

1. Taschenrechner für die Voraussage periodischer Ereignisse folgende Teile umfassend: eine elektrische Speisung, Eingangsmittel (2), welche die Mitteilung von Daten mit Datumangaben und Rechenbefehle gestatten, mindestens einen Ausgang umfassen und an diesem Ausgang die besagten Daten und Befehle als elektrische Signale liefern, elektronische Rechenmittel (50) mit Eingängen für die besagten Signale, mit derart angeordnete Rechen- (50) und Speicher- (60, 62, 80) Schaltkreise, dass sie, als Antwort auf Befehle, die den Eingangsmitteln mitgeteilt sind, Voraussagerechnungen durchführen, und mit mindestens einem Ausgang (57) für die den abgefragten Ergebnissen entsprechenden Ausgangssignale, dadurch gekennzeichnet, dass er zusätzlich, um die wichtigsten variablen Daten zu bewahren, einen elektromechanischen Speicher (80) umfasst, der aus elektromechanischen Speichereinheiten besteht, wovon jede als bistabiles elektromechanisches Miniaturrelais (82) besteht, das mindestens ein Steuersolenoid (83, 84) umfasst und selbst bei fehlender elektrischer Speisung seine Stellung behält, derart, dass die wichtigsten variablen Daten sogar bei unterbrochener Speisung geschützt sind, wobei die Relaiskontakte der Ablesung der durch das Relais gebildeten Speichereinheit dienen und der Speicher (80) Schreibschaltkreise (72) umfasst mit welchen auf Wunsch ein Impuls zu jedem Solenoid (82) übertragen werden kann, und Leseschaltkreise (78) umfasst, mit welchen auf Wunsch der Zustand, offen oder geschlossen, von jedem Kontakt geprüft werden kann, wobei die Leseschaltkreise von den Rechenmitteln gesteuert sind, und dass der elektromechanische Speicher (80) derart angeordnet ist, dass er nur von den Rechenmitteln (50) gelesen wird und dass er durch einander folgende Dateneingaben mittels Handbetätigung der Eingangsmittel (2) auf den neuen Stand gebracht wird und dass für die Eingebung der Daten in den elektromechanischen Speicher, der Taschenrechner eine Steuertaste (29) und eine Sicherheitstaste (28) umfasst, die derart angeordnet sind, dass um Daten in den elektromechanischen Speicher (80) einzugeben, beide Tasten (28, 29) gedrückt werden müssen.

2. Rechner nach Anspruch 1, dadurch gekennzeichnet, dass er eine Zeitbasis und elektronische logische Zeitmessschaltungen enthält und dass die Rechen- und Anzeigemittel Zeit- Mess- und -Anzeigefunktionen enthalten.

3. Rechner nach Anspruch 1 oder 2, dadurch gekennzeichnet, dass die Anzeige mehrere elektrooptische Einheiten umfasst, um das Rechenresultat anzuzeigen.

4. Rechner nach Anspruch 1, dadurch gekennzeichnet, dass die Anzeige mehrere elektrisch leuchtende Einheiten mit verschiedenen Farben umfasst.

5. Rechner nach Ansprüche 1 oder 2, dadurch gekennzeichnet, dass die Anzeigemittel eine numerische Anzeige umfassen, welche mindestens erlaubt, die eingegebenen Daten zu lesen.

6. Rechner nach einem der vorangehenden Ansprüche, dadurch gekennzeichnet, dass die Anzeigemittel eine alphanumerische Anzeige umfassen.

7. Rechner nach einem der vorangehenden Ansprüche, dadurch gekennzeichnet, dass der elektromechanische Speicher (80) durch eine Adapterschaltung (70) mit einem zentralen Bus (52) verbunden ist.

8. Rechner nach Anspruch 7, dadurch gekennzeichnet, dass die Adapterschaltung (70) einen Multiplexer (702) umfasst.

**Claims**

1. A pocket calculator for predicting periodic events comprising: an electric power supply, entry means (2) for entering data covering dates and instructions for carrying out the calculations, said means comprising at least one output through which said data and said instructions are delivered as electric signals, electronic computer means (50) comprising an input for said signals, computer circuits (50) and memories (60, 62, 80) arranged to carry out the prospective calculations in response to instructions received through the entry means and at least one output (57) for transmitting the signals corresponding to the results of such calculations, and display means (3); characterized in that it comprises for storing the relevant essential data an electro-mechanical memory (80), each unit of the electro-mechanical memory consisting of a miniaturized bistable electro-mechanical relay (82) provided with at least one control solenoid (83, 84) and which retains its mechanical position as means of storing essential variable data even in the event of the electric power supply being interrupted, the contactors of the relay enabling the reading of the memory unit (82), the memory (80) further comprising entry circuits (72) for sending when desired impulses to each solenoid (82) and reader circuits (78) for checking the status of each contactor (i.e. closed or open), said reader circuits being controlled by the computer means (50); further characterized in that the electro-mechanical memory (80) is arranged so that it can be read only by the computer means (50) and so that its contents can be updated by the successive introductions of data by manually operating the entry means (2), and in that the calculator comprises a control key (29) and a safety key (28) which must be both pressed before data can be entered into the electro-mechanical memory (80).

2. A calculator according to claim 1, further characterized in that it comprises a time base and electronic logical circuits for measuring time, and that the computer means and display means comprise means for measuring and displaying the time.

3. A calculator according to claim 1 or 2, further characterized in that the display means comprise a plurality of electro-optical elements for displaying the results of the calculations.

4. A calculator according to claim 1, further characterized in that the display means comprise a plurality of electroluminescent elements of different colours.

5. A calculator according to claim 1 or 2, further characterized in that the display means enable the readout of at least the data which are entered.

6. A calculator according to one of the preceding claims further characterized in that the readout on the display means is alphanumeric.

7. A calculator according to one of the preceding claims, further characterized in that the electro-mechanical memory (80) is connected to a central bus (52) by an interface circuit (70).

8. A calculator according to claim 7, further characterized in that the interface circuit (70) comprises a multiplexer (702).

FIG.1

FIG.5

FIG. 2

FIG. 4

FIG.3

FIG.8

FIG.6

FIG.7

4

FIG.9